# EUROPEAN PATENT SPECIFICATION

(11) **EP 4 022 083 B1**
(45) Date of publication and mention of the grant of the patent: **03.07.2024**
(21) Application number: 20768871.4
(22) Date of filing: 28.08.2020
(51) Int. Cl.: C12Q 1/6806

(54) **COMPOSITIONS AND METHODS FOR MULTIPLEX RT-PCR AND GENETIC ANALYSIS**
ZUSAMMENSETZUNGEN UND VERFAHREN FÜR MULTIPLEX-PCR UND GENETISCHEN ANALYSE
COMPOSITIONS ET PROCÉDÉS POUR RT-PCR MULTIPLEX ET ANALYSE GÉNÉTIQUE

(30) Priority: 30.08.2019 US 201962893974 P
(43) Date of publication of application: 06.07.2022
(73) Proprietor: Life Technologies Corporation, Carlsbad, CA 92008 (US)
(72) Inventor: CHEN, Sihong, Carlsbad, California 92008 (US)
(74) Representative: Thermo Fisher Scientific- Life Sciences Solutions Group
(86) International application number: PCT/US2020/070471
(87) International publication number: WO 2021/042129

(56) References cited:
- EP-A2- 1 050 587
- WO-A1-2013/024064
- US-A- 6 015 376

## Description

### FIELD OF THE INVENTION

The present invention is directed to compositions and methods useful for the generation of nucleic acids from a ribonucleic acid template and further nucleic acid amplification. Specifically, the invention is directed to the generation and amplification of nucleic acids by reverse transcriptase-polymerase chain reaction (RT-PCR).

### BACKGROUND OF THE INVENTION

Reverse transcription of RNA, followed by polymerase chain reaction amplification, commonly referred to as RT-PCR, is widely used for the detection and quantification of RNA. Success of reverse transcription however is highly dependent upon reaction components and conditions and reactions should be carried out appropriately and effectively to fit with downstream applications of interest. References such as EP1050587, WO2013/024064 and US6015376 disclose the generation and amplification of nucleic acids by RT-PCR with various additives added in order to optimize the RT-PCR reaction. Although improved methods have been developed that successfully demonstrate significant reduction of inhibitory effects of reverse transcriptase on subsequent polymerase amplification, further improved specificity and sensitivity of RT-PCR by more effective approaches are still a need in the art.

### DESCRIPTION OF THE INVENTION

The invention is useful in the rapid efficient production and amplification of cDNAs (single-stranded and double-stranded) for detection and analysis of RNA molecules, which may be used for a variety of research, industrial, medical and forensic purposes. The features of the invention are set forth with particularity in the appended claims. A better understanding of the features and advantages of the present invention will be obtained by reference to the following detailed description that sets forth illustrative embodiments, in which the principles of the invention are utilized.

Provided in the instant invention are compositions and methods for efficient procedures which enable rapid preparation of RNA to cDNA for multiplexed libraries suitable for downstream analysis. Certain methods comprise streamlined, addition-only procedures conveying effective library generation for genetic analysis.

In one aspect of the invention, compositions and methods are provided that are suitable for reverse transcription polymerase chain reaction (RT-PCR) of RNA. Such compositions comprise a mixture of oligonucleotide primer nucleic acids or derivatives thereof, a buffering agent comprising Tris, a magnesium chloride salt, an ammonium sulfate salt, deoxyribonucleoside triphosphates or derivatives thereof, and additives useful in RT-PCR. Methods of use of provided compositions are further provided.

In some examples, compositions comprise oligonucleotide primer nucleic acids comprising any of random primers, primers specific to a target RNA template, and/or homopolymer primers and/or a mixture of two or more of the foregoing. In certain examples the primers are random hexamer oligonucleotide primers. In certain examples the primers are target specific primers. In certain examples the primers are homopolymeric oligonucleotide primers. In some examples at least 10ng of oligonucleotide primer or at least 100 ng/mL of oligonucleotide primer is present. In some examples between about 10ng of oligonucleotide primer to about 20000 ng of oligonucleotide primer or between about 100ng/mL of oligonucleotide primer to about 10mg/mL oligonucleotide primer is present. In some examples provided compositions further comprise one or more additional oligonucleotide primers, the primers preferably being random primers, homopolymers, and/or primers specific to a target RNA template.

In some instances, compositions of the invention further comprise at least one member of a reverse transcriptase enzyme. In some examples at least one or more reverse transcriptase is selected from AMV-RT, M-MLV-RT, HIV-RT, EIAV-RT, RAV2-RT, C. hydrogenoformans DNA Polymerase, SuperScript I, SuperScript II, SuperScript III, SuperScript IV and/or mutants, variants and derivatives thereof. In particular examples, the reverse transcriptase is SuperScript IV.

In some examples compositions of the invention further comprise at least one member of a DNA polymerase enzyme. In some examples at least one or more the DNA polymerase is Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT^{™}, DEEPVENT^{™} DNA polymerase, and/or mutants, variants and derivatives thereof.

In some examples compositions of the invention include a buffering agent, comprising Tris at pH of about 8 to about 9.

In some examples compositions of the invention further comprise an additive useful in RT-PCR preferably being DMSO, DTT, glycerol, formamide, betain, tetramethylammonium chloride, PEG, Tween 20, NP 40, ectoine, polyoles, E. coli SSB protein, Phage T4 gene 32 protein, and/or BSA.

In some provided examples, compositions comprise nucleotide triphosphates selected from dATP, dCTP, dGTP, dTTP, dITP, dUTP, α-thio-dNTPs, biotin-dUTP, fluorescein-dUTP, and/or digoxigenin-dUTP. In particular examples, the nucleotide triphosphates are deoxyribonucleoside triphosphates selected from the group consisting of dATP, dTTP, dGTP, and dCTP.

In some examples, provided compositions comprise a mixture of random hexamer primer nucleic acids, a buffering agent comprising Tris, a magnesium chloride salt, an ammonium sulfate salt, deoxyribonucleoside triphosphates, DTT, and glycerol. In certain examples compositions comprise about 24ng/uL random hexamer, about 25mM Tris, about 4mM magnesium chloride, about 10mM ammonium sulfate, about 1mM dNTP, about 1mM DTT, about 4.4% glycerol and optionally reverse transcriptase. In other certain examples compositions comprise about 120ng/uL random hexamer, about 125mM Tris, about 20mM magnesium chloride, about 50 ammonium sulfate, about 5mM dNTP, about 5mM DTT, about 22% glycerol.

Further provided are reagent kits for use in RT-PCR comprising compositions of the invention. In some examples a reverse transcriptase, and optionally a DNA polymerase and are combined in one container, and a mixture of random hexamer primer nucleic acids, a buffering agent comprising Tris, a magnesium chloride salt, an ammonium sulfate salt, deoxyribonucleoside triphosphates, DTT, and glycerol are combined in a second container. In some examples a reverse transcriptase, and optionally a DNA polymerase and are combined in one container, and a mixture of random hexamer primer nucleic acids, a buffering agent comprising Tris, a magnesium chloride salt, an ammonium sulfate salt, deoxyribonucleoside triphosphates, DTT, and glycerol are combined in a second container. In certain examples a kit comprises SuperScript IV RT Enzyme mix in one container and a mixture comprising about 120ng/uL random hexamer, about 125mM Tris, about 20mM magnesium chloride, about 50 ammonium sulfate, about 5mM dNTP, about 5mM DTT, about 22% glycerol in a second container.

In some aspects, use of a composition of the invention or use of a reagent kit according to the invention for RT-PCR is provided.

In one aspect of the invention, methods are provided that are suitable for reverse transcription polymerase chain reaction (RT-PCR) of RNA. Provided methods comprise providing a sample comprising RNA template to a reaction mixture comprising oligonucleotide primer nucleic acids or derivatives thereof, a buffering agent comprising Tris, a magnesium chloride salt, an ammonium sulfate salt, deoxyribonucleoside triphosphates or derivatives thereof, and additives useful in RT-PCR; mixing the reaction mixture with reverse transcriptase, and incubating the reaction mixture under conditions sufficient to allow polymerization of a nucleic acid molecule complementary to a portion of the RNA template. In some examples, methods comprise use of oligonucleotide primer nucleic acids comprising any of random primers, primers specific to a target RNA template, and/or homopolymer primers and/or a mixture of two or more of the foregoing. In certain examples the primers are random hexamer oligonucleotide primers. In certain examples the primers are target specific primers. In certain examples the primers are homopolymeric oligonucleotide primers. In some examples at least 10ng of oligonucleotide primer or at least 100 ng/mL of oligonucleotide primer is present. In some examples between about 10ng of oligonucleotide primer to about 20000 ng of oligonucleotide primer or between about 100ng/mL of oligonucleotide primer to about 10mg/mL oligonucleotide primer is present. In some examples provided compositions and methods further comprise one or more additional oligonucleotide primers, the primers preferably being random primers, homopolymers, and/or primers specific to a target RNA template.

In some examples methods of the invention comprise use of at least one member of a reverse transcriptase enzyme. In some examples at least one or more reverse transcriptase is selected from AMV-RT, M-MLV-RT, HIV-RT, EIAV-RT, RAV2-RT, C. hydrogenoformans DNA Polymerase, SuperScript I, SuperScript II, SuperScript III, SuperScript IV and/or mutants, variants and derivatives thereof. In particular examples, the reverse transcriptase is SuperScript IV.

In some examples methods of the invention further comprise use of at least one member of a DNA polymerase enzyme. In some examples a DNA polymerase is provided with a reverse transcriptase mixture. In some examples a DNA polymerase is provided in the reaction mixture. In some examples at least one or more the DNA polymerase is Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT^{™}, DEEPVENT^{™} DNA polymerase, and/or mutants, variants and derivatives thereof.

In some examples compositions used in the methods of the invention include a buffering agent, comprising Tris at pH of about 8 to about 9.

In some examples a reaction compositions used in the methods of the invention further comprise an additive useful in RT-PCR preferably being DMSO, DTT, glycerol, formamide, betain, tetramethylammonium chloride, PEG, Tween 20, NP 40, ectoine, polyoles, E. coli SSB protein, Phage T4 gene 32 protein, and/or BSA.

In some provided aspects, reaction composition used in the method comprises nucleotide triphosphates preferably are selected from dATP, dCTP, dGTP, dTTP, dITP, dUTP, α-thio-dNTPs, biotin-dUTP, fluorescein-dUTP, and/or digoxigenin-dUTP. In particular xamplese, the nucleotide triphosphates are deoxyribonucleoside triphosphates selected from the group consisting of dATP, dTTP, dGTP, and dCTP.

In some examples, provided methods comprise use of a reaction composition comprising a mixture of random hexamer primer nucleic acids, a buffering agent comprising Tris, a magnesium chloride salt, an ammonium sulfate salt, deoxyribonucleoside triphosphates, DTT, and glycerol. In certain examples methods comprise use of a reaction composition comprising about 24ng/uL random hexamer, about 25mM Tris, about 4mM magnesium chloride, about 10mM ammonium sulfate, about 1mM dNTP, about 1mM DTT, about 4.4% glycerol and optionally reverse transcriptase. In other certain examples methods comprise use of a composition comprising about 120ng/uL random hexamer, about 125mM Tris, about 20mM magnesium chloride, about 50 ammonium sulfate, about 5mM dNTP, about 5mM DTT, about 22% glycerol.

In some examples provided methods further comprise amplification of the resulting cDNA molecule complementary to a portion of the RNA template by a process of DNA replication comprising a polymerase chain reaction (PCR). In certain examples provided methods are carried out wherein the reactions of DNA generation and amplification occurs in an addition only uncoupled reaction mixture.

In some examples methods comprise a reverse transcription reaction occurs between about 35°C to about 70°C and the DNA replication comprises PCR amplification thermocycling. In some examples PCR amplification comprises at least 2 or 3 cycles of denaturation, annealing, and polymerization. In some examples PCR amplification comprises at least 15 cycles of denaturation, annealing, and polymerization. In some examples PCR amplification comprises multiplex amplification. In certain examples multiplex PCR amplification comprises multiple different PCR amplification rounds.

Section headings used herein are for organizational purposes only and are not to be construed as limiting the described subject matter in any way. When definitions of terms in cited references appear to differ from the definitions provided in the present teachings, the definition provided in the present teachings shall control. It will be appreciated that there is an implied "about" prior to the temperatures, concentrations, times, etc discussed in the present teachings, such that slight and insubstantial deviations are within the scope of the present teachings herein. In this application, the use of the singular includes the plural unless specifically stated otherwise. It is noted that, as used in this specification, singular forms "a," "an," and "the," and any singular use of a word, include plural referents unless expressly and unequivocally limited to one referent. Also, the use of "comprise", "comprises", "comprising", "contain", "contains", "containing", "include", "includes", and "including" are not intended to be limiting. It is to be understood that both the general description is exemplary and explanatory only and not restrictive of the invention.

Unless otherwise defined, scientific and technical terms used in connection with the invention described herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization used herein are those well-known and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. Techniques and procedures described herein are generally performed according to conventional methods well known in the art and as described in various general and more specific references that are cited and discussed throughout the instant specification. *See, e.g.*, Sambrook et al., Molecular Cloning: A Laboratory Manual (Third ed., Cold Spring Harbor Laboratory Press, Cold Spring Harbor, N.Y. 2000). Unless specifically provided, any nomenclature utilized in connection with, and laboratory procedures and techniques described herein are those well-known and commonly used in the art.

### Reverse Transcriptase-Polymerase Chain Reaction (RT-PCR)

RT-PCR is an established molecular methodology used to generate and replicate a nucleic acid derived from an RNA template. RT-PCR is described herein as an exemplary protocol capable of utilizing provided compositions and methods of the present invention without limitation. RT-PCR involves two separate molecular syntheses: (i) synthesis of cDNA from RNA template; and (ii) replication of the newly synthesized cDNA through PCR amplification. It will be understood by one skilled in the art that the provided compositions and methods of the invention have utility in other processes, which involve combination of reverse transcriptase and DNA polymerase activity in conjunction with multiplex amplifications.

In RT-PCR, a reaction mixture is first incubated (in an appropriate buffering agent) at a temperature sufficient to allow synthesis of a DNA molecule complementary to at least a portion of an RNA template. Components of a reverse transcription reaction mixture typically include an RNA template, from which the complementary DNA (cDNA) is transcribed; a nucleic acid polymerase that exhibits reverse transcriptase activity; and the appropriate nucleotide building blocks needed for nucleic acid synthesis. For the purposes of this invention, cDNA is defined as any DNA molecule whose nucleic acid sequence is complementary to an RNA molecule. An RNA template is defined as any RNA molecule used to provide a nucleic acid sequence from which a cDNA molecule may be synthesized. The synthesis of cDNA from an RNA template is typically accomplished by utilizing a nucleic acid polymerase that exhibits reverse transcriptase activity. For the purposes of this invention, reverse transcriptase activity refers to the ability of an enzyme to polymerize a cDNA molecule from an RNA template, and reverse transcriptase broadly refers to any enzyme possessing reverse transcriptase activity. Reverse transcription typically occurs in a temperature range from about 20°C to about 75°C, preferably from about 35°C to about 70°C.

After reverse transcription of an RNA template to produce a cDNA molecule, the cDNA is incubated (in an appropriate buffering agent) under conditions sufficient for replication of the cDNA molecule. The reaction mixture may be the same as that of the previous reverse transcription reaction mixture, as employed in coupled (also called continuous, or one-step) RT-PCR, or the reaction mixture may comprise an aliquot of the previous reverse transcription reaction mixture and may be further modified for nucleic acid replication, as in uncoupled (or two-step) RT-PCR. In particularly preferred examples, an uncoupled RT-PCR is employed comprising a multiplex PCR for further analysis (e.g., sequencing, e.g., NGS sequencing applications). Components of a replication reaction mixture typically include a nucleic acid template (i.e., cDNA, DNA); a nucleic acid polymerase; and appropriate nucleotide building blocks needed for nucleic acid synthesis. Nucleic acid replication refers to polymerization of a nucleic acid whose sequence is determined by, and complementary to, another nucleic acid. DNA replication, as used herein, is synonymous with DNA amplification. Preferably DNA amplification occurs repetitively, thus replicating both strands of each nucleic acid sequence, i.e., DNA complementary to the RNA template, and DNA whose nucleic acid sequence is substantially identical to the original RNA template. Repetitive, or cyclic, DNA replication may be advantageously accomplished using a thermostable polymerase in a Polymerase Chain Reaction (PCR).

PCR is a technique well known in the art. PCR is used to amplify nucleic acids by subjecting a reaction mixture to cycles of (i) nucleic acid denaturation, (ii) oligonucleotide primer annealing, and (iii) nucleic acid polymerization. Preferred reaction conditions for amplification comprise thermocycling, i.e., alternating the temperature of the reaction mixture to facilitate each of the steps of the PCR cycle. PCR is typically extended through multiple cycles of denaturation, annealing and replication, augmented (optionally and preferably) with an initial prolonged denaturation step and a final prolonged extension (polymerization) step. Thermocycling typically occurs within a temperature range of between about 23°C to about 100°C, and preferably between about 37°C to about 99°C. Nucleic acid denaturation typically occurs between about 90°C to about 100°C, preferably about 90°C. Annealing typically occurs between about 37°C to about 75°C, preferably about 60°C to about 65°C. Polymerization typically occurs between about 55°C to about 80°C, preferably about 72°C. The number of thermocycles varies immensely, depending upon practitioner preference and quantity of DNA product desired and application. Preferably, the number of PCR cycles ranges from about 5 to about 99, more preferably greater than about 20 cycles, most preferably about 40 cycles. In some examples multiple rounds of multiplex PCR amplifications are carried out. In certain examples multiplex amplification is carried out according to the methods outlined in ION AMPLISEQ library preparation technology according to the manufacturer instructions or in variations thereof however keeping with the original intent and scope of the technology. In other certain examples multiplex amplification is carried out according to methods outlined in ION AMPLISEQ HD library preparation technology according to the manufacturer instructions, or in variations thereof, however keeping with the original intent and scope of the technology. In still other certain examples additional multiplex library preparation technology (e.g., rhAmpSeq, AmpliSeq for Illumina, Accel amplicon, SUREMastr, QIAseq, NEXTflex, AMP Archer Assays, CleanPlex, NEBNext, EasySeq, NEXTERA, etc. may be implemented using commercially available products and kits and carrying out multiplex preparations according to manufacturer instructions, or variations thereof, however keeping with the original intent and scope of the technology. In one example multiplex PCR amplification is carried out using ION AMPLISEQ technology: For example, in a method of the invention, a first RT reaction is carried out in a reaction comprising 1x RT SuperScriptIV (VILO) enzyme mix, 1x URT reaction buffer as provided herein, and 20ng TNA or 10ng RNA. cDNA is prepared by incubating the reaction for 10 minutes at 25*C followed by 10 minutes at 50*C then the reaction is inactivated for 5 minutes at 85*C followed by a 4*C hold. Library cDNA can then be prepared using a preferred ION AMPLISEQ library preparation products and protocols and/or ONCOMINE library preparation products and protocols. For example, a primer panel is combined with prepared cDNA reaction mix, forward primer, reverse primer, and enzyme master mix; the reaction is amplified using PCR amplification for about 15 to about 32 cycles. Amplified products are then partially digested with FUPA reagent, followed by ligation of universal primers to resulting amplicons. A further library amplification by PCR using universal primers (e.g., about 15 to about 18 cycles) is carried out before preparation for templating and sequencing. In one example multiplex PCR amplification is carried out using ION AMPLISEQ HD technology: For example, in a method of the invention, a first RT reaction is carried out in a reaction comprising 1x RT SuperScriptIV (VILO) enzyme mix, 1× URT reaction buffer as provided herein, and 20ng TNA or 10ng RNA. cDNA is prepared by incubating the reaction for 10 minutes at 25*C followed by 10 minutes at 50*C then the reaction is inactivated for 5 minutes at 85*C followed by a 4*C hold. Library cDNA can then be prepared using a preferred ION AMPLISEQ HD library preparation products and protocols and/or ONCOMINE HD library preparation products and protocols. For example, a primer panel is combined with prepared cDNA reaction mix, forward primer, reverse primer, and amplification enzyme master mix; the reaction is amplified using PCR amplification for about 2 to about 5 initial tagging cycles. Amplified products are then partially digested with SUPA reagent, followed by a second amplification of about 18 to about 30 cycles using universal primers to prepare final amplicons before purification and normalization in preparation for templating and sequencing. In certain examples, prepared libraries are further processes for nucleic acid sequencing analysis. In particular examples prepared libraries are sequenced using ION TORRENT sequencing systems. In particular examples prepared libraries are sequenced using ILLUMINA sequencing systems. In still other examples, other commercially available technology platforms for multiplex amplification and preparation of libraries for nucleic acid analyses, including but not limited to sequencing, and particularly next generation sequencing (NGS) are contemplated. One skilled in the art would understand and contemplate alternative methods will be utilized in conjunction with provided compositions and methods of the invention, which are also encompassed within the scope of the instant invention.

### Template RNA

Template RNA can be any ribonucleic acid of interest, known (or unknown) to the practitioner in a sample. Template RNA can be artificially synthesized or isolated from natural sources. In some examples, RNA template can be a ribonucleic acid such as RNA, mRNA, piRNA, tRNA, rRNA, ncRNA, shRNA, siRNA, snRNA, miRNA, snoRNA and viral RNA. Preferably, the RNA is mRNA. More preferably RNA is biologically active or encodes or partially encodes a biologically active polypeptide, or mutant thereof. RNA can be from any prepared sample such as a synthetic sample or a biological sample. In some examples a sample includes cell-free nucleic acids from a biological fluid (e.g., blood, serum, urine, sputum, saliva, etc.), nucleic acids from a tissue, nucleic acids from a biopsied tissue, nucleic acids from a needle biopsy, nucleic acids from a single cell or nucleic acids from two or more cells. In some examples a plurality of initial polynucleotides comprises a formalin fixed paraffin-embedded (FFPE) sample; genomic DNA and/or RNA; cell free DNA and/or RNA; circulating tumor DNA and/or RNA; fresh frozen sample, or a mixture of two or more of the foregoing; and in some embodiments a plurality of initial polynucleotides comprises a nucleic acid reference standard. In some examples, a sample includes nucleic acid molecules obtained from biopsies, tumors, scrapings, swabs, blood, mucus, urine, plasma, semen, hair, laser capture micro-dissections, surgical resections, and other clinical or laboratory obtained sample. In some examples, a sample is an epidemiological, agricultural, forensic or pathogenic sample. In certain examples, a sample includes a reference. In some examples a sample is a normal tissue or well documented tumor sample. In certain examples a reference is a standard nucleic acid sequence (e.g., Hg19).

An RNA template can be present in any useful amount. In some examples, the RNA template concentration is 50 pg/µL to 5ng/uL or less. One of skill in the art will appreciate that other RNA template concentrations are useful in the present invention. Suggested input quantities of template are: 0.1 pg to 100 ng total RNA; 10 fg to 100 ng polyA(+) RNA. In particular examples input quantity of template is about 10 ng RNA and/or about 20ng TNA from cell free sample.

### Reverse Transcriptase

Reverse transcriptases useful in the present invention may be any polymerase that exhibits reverse transcriptase activity. Several reverse transcriptases are known in the art and are commercially available. Preferred enzymes include those that exhibit reduced RNase H activity. Preferred reverse transcriptases include: Avian Myeloblastosis Virus reverse transcriptase (AMV-RT), Moloney Murine Leukemia Virus reverse transcriptase (MMLV-RT), Human Immunovirus reverse transcriptase (HIV-RT), EIAV-RT, RAV2-RT, C. hydrogenoformans DNA Polymerase, rTth DNA polymerase, SuperScript I, SuperScript II, Superscript III, SuperScript IV, and mutants, variants and derivatives thereof. In certain examples enzymes for use in the invention include, but are not limited to, M-MLV reverse transcriptase (RNase H- or substantially reduced in RNase H activity), and RSV reverse transcriptase Rnase- and mutants, variants or derivatives thereof. In particular examples reverse transcriptase is Superscript III, SuperScript IV, or mutants, variants or derivatives thereof. In particular examples, the reverse transcriptase is SuperScript IV. It is to be understood that a variety of reverse transcriptases may be used in the present invention, including reverse transcriptases not specifically disclosed above, without departing from the scope or preferred examples thereof.

### DNA Polymerases

DNA polymerases useful in the present invention may be any polymerase capable of replicating a DNA molecule. Preferred DNA polymerases are thermostable polymerases, which are especially useful in PCR. Thermostable polymerases are isolated from a wide variety of thermophilic bacteria, such as Thermus aquaticus (Taq), Thermus brockianus (Tbr), Thermus flavus (Tfl), Thermus ruber (Tru), Thermus thermophilus (Tth), Thermococcus litoralis (Tli) and other species of the Thermococcus genus, Thermoplasma acidophilum (Tac), Thermotoga neapolitana (Tne), Thermotoga maritima (Tma), and other species of the Thermotoga genus, Pyrococcus furiosus (Pfu), Pyrococcus woesei (Pwo) and other species of the Pyrococcus genus, Bacillus sterothermophilus (Bst), Sulfolobus acidocaldarius (Sac) Sulfolobus solfataricus (Sso), Pyrodictium occultum (Poc), Pyrodictium abyssi (Pab), and Methanobacterium thermoautotrophicum (Mth), and mutants, variants or derivatives thereof.

Several DNA polymerases are known in the art and are commercially available. In some examples a thermostable DNA polymerase is selected from the group of Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT^{™}, DEEPVENT^{™}, and active mutants, variants and derivatives thereof. In some examples, the DNA polymerase is Taq DNA polymerase or active mutants, variants, and derivatives thereof. It is to be understood that a variety of DNA polymerases and/or combinations thereof may be used in the present invention, including DNA polymerases not specifically disclosed above, without departing from the scope or preferred embodiments thereof.

The reverse transcriptase can be present in any appropriate ratio to the DNA polymerase. in particularly preferred compositions the unit ratio of the reverse transcriptase enzymes to the DNA polymerase enzymes ranges from about 0.2:2 to about 500:2, preferably from about 0.5:2 to about 250:2 In some examples, the ratio of reverse transcriptase to DNA polymerase in unit activity is greater than or equal to 3. One of skill in the art will appreciate that other reverse transcriptase to DNA polymerase ratios are useful in the present invention.

Thermostable DNA polymerase is added to the present compositions at a final concentration in solution of about 0.1-200 units microliter, about 0.150 units per micro, about 0.1-100 units per microliter, about 0.1-50 units per microliter, about 0.1-40 units per microliter, about 0.25-30 units per microliter, about 0.5-25 units per microliter, or about 0.5-20 units per microliter, or at a concentration of about 1 to about 10 units per microliter.

### Oligonucleotide Primers

Provided compositions and methods of use thereof comprise one or more primers which facilitate the synthesis of a first DNA molecule complementary to all or a portion of an RNA template (e.g., a single-stranded cDNA molecule). Primers may also be used to synthesize a DNA molecule complementary to all or a portion of the first DNA molecule, thereby forming a double-stranded cDNA molecule. Additionally, primers may be used in amplifying nucleic acid molecules in accordance with the invention. Additional and/or alternative primers that may be used for amplification of the DNA molecules according to the methods of the invention will be apparent to one of ordinary skill in the art. Oligonucleotide primers useful in the present invention can be any oligonucleotide of two or more nucleotides in length. Preferably, PCR primers are about 5 to about 30 bases in length, and are not palindromic (self-complementary) or complementary to other primers that can be used in the reaction mixture. Primers can be, but are not limited to, random or arbitrary primers, homopolymers, or primers specific to a target RNA template (e.g., a sequence specific primer). Oligonucleotide primers are oligonucleotides used to hybridize to a region of a target nucleic acid to facilitate the polymerization of a complementary nucleic acid. In preferred RT-PCR techniques, primers serve to facilitate reverse transcription of a first nucleic acid molecule complementary to a portion of an RNA template (e.g., a cDNA molecule), and also to facilitate replication of the nucleic acid (e.g., PCR amplification of DNA). Any primer can be synthesized by a practitioner of ordinary skill in the art or can be purchased from any of a number of commercial vendors. It is to be understood that a vast array of primers can be useful in the present invention, including those not specifically disclosed herein, without departing from the scope or preferred embodiments thereof.

In some examples, compositions comprise oligonucleotide primer nucleic acids comprising any of random primers, primers specific to a target RNA template, and/or homopolymer primers and/or a mixture of two or more of the foregoing. In certain examples the primers are random hexamer oligonucleotide primers. In certain examples the primers are target specific primers. In certain examples the primers are homopolymeric oligonucleotide primers. In some examples at least 10ng of oligonucleotide primer or at least 100 ng/mL of oligonucleotide primer is present. In some examples between about 10ng of oligonucleotide primer to about 20000 ng of oligonucleotide primer or between about 100ng/uL of oligonucleotide primer to about 10mg/mL oligonucleotide primer is present. In particular examples primers comprise random hexamer present at a concentration between about 50ng/uL to about 150ng/uL In other particular examples primers comprise random hexamer present at a concentration between about 10ng/uL to about 15ng/uL. In some examples provided compositions further comprise one or more additional oligonucleotide primers, the additional primers preferably being random primers, homopolymers, and/or primers specific to a target RNA template.

### Nucleotide Bases

Provided compositions of the invention and method of use thereof comprise one or more nucleotides (e.g., deoxynucleoside triphosphates (dNTPs)). Nucleotide bases useful in the present invention may be any nucleotide useful in the polymerization of a nucleic acid. Nucleotides may be naturally occurring, unusual, modified, derivative, or artificial. Nucleotides may be unlabeled, or detectably labeled by methods known in the art (e.g., using radioisotopes, vitamins, fluorescent or chemiluminescent moieties, dioxigenin). Preferably the nucleotides are deoxynucleoside triphosphates, dNTPs (e.g., dATP, dCTP, dGTP, dTTP, dITP, dUTP, α-thio-dNTPs, biotin-dUTP, fluorescein-dUTP, digoxigenin-dUTP, 7-deaza-dGTP). dNTPs are also well known in the art and are commercially available.

Nucleotides comprises in the compositions and methods of the present invention can be present in any concentration. In some examples, the nucleotides is present in an amount from about 1 µM to about 1000 µM. In other examples, the nucleotides is present in an amount from about 10 µM to about 750 µM. In still other examples, the nucleotides is present in an amount from about 100 µM to about 500 µM. One of skill in the art will appreciate that other concentrations of nucleotides are useful in the present invention.

### Buffering Agents and Salt Solutions

Buffering agents and salts useful in the present invention provide appropriate stable pH and ionic conditions for nucleic acid synthesis, e.g., for reverse transcriptase and DNA polymerase activity. A wide variety of buffers and salt solutions and modified buffers are known in the art that can be useful in the present invention, including agents not specifically disclosed herein. Preferred buffering agents include, but are not limited to, TRIS, TRICINE, BIS-TRICINE, HEPES, MOPS, TES, TAPS, PIPES, CAPS. In a preferred embodiment, provided compositions include TRIS. In some embodiments compositions used in the methods of the invention include a buffering agent, comprising TRIS at pH of about 8 to about 9, and in certain preferred embodiments TRIS is TRIS-HCl (pH 8.0-9.0). Preferred salt solutions include, but are not limited to solutions of, ammonium sulfate, magnesium chloride, potassium acetate, potassium sulfate, potassium chloride, ammonium chloride, ammonium acetate, magnesium acetate, magnesium sulfate, manganese chloride, manganese acetate, manganese sulfate, sodium chloride, sodium acetate, lithium chloride, and lithium acetate. In a preferred examples provided compositions include ammonium sulfate and magnesium chloride. In certain preferred examples provided compositions include TRIS-HCl, ammonium sulfate and magnesium chloride.

Buffering agents comprised in the compositions of the present invention can be present in any concentration. In some examples, the buffer is present in an amount from about 0.1 mM to about 1000 mM. In other examples, the buffer is present in an amount from about 1 mM to about 500 mM. In still other examples, the buffer is present in an amount from about 5 mM to about 250 mM. One of skill in the art will appreciate that other concentrations of buffer are useful in the present invention.

Salts comprises in the compositions the present invention can be present in any concentration. In some examples, the salt is present in an amount from about 0.01 mM to about 1000 mM. In other examples, the salt is present in an amount from about 0.1 mM to about 500 mM. In still other examples, the salt is present in an amount from about 1 mM to about 100 mM. One of skill in the art will appreciate that other concentrations of salts are useful in the present invention.

In certain preferred examples provided compositions include TRIS-HCl at about 125mM, ammonium sulfate at about 50mM and magnesium chloride at about 20mM. In certain preferred examples provided compositions include TRIS-HCl at about 25mM, ammonium sulfate at about 10mM and magnesium chloride at about 4mM. One of skill in the art will appreciate that other concentrations of buffers and salts are useful in the present invention.

### Other Additives useful in RT-PCR

Other additives capable of facilitating reverse transcription, replication, and/or a combination of both reactions (e.g., agents for facilitating RT-PCR), are known in the art. In accordance with the compositions and methods of this invention, one or more of these additives can be incorporated in the present compositions to optimize the generation and replication of nucleic acids from a ribonucleic acid template. Additives can be organic or inorganic compounds. Inhibition-relieving agents useful in the present invention include, but are not limited to, polypeptides such as; human serum albumin, bovine serum albumin (BSA), ovalbumin, albumax, casein, gelatin, collagen, globulin, lysozyme, transferrin, myoglobin, hemoglobin, α-lactalbumin, fumarase, glyceraldehyde-3 -phosphate dehydrogenase (GAPDH), amyloglucosidase, carbonic anhydrase, β-lactoglobulin, aprotinin, soybean trypsin inhibitor, trypsinogen, phosphorylase b, myosin, actin, β -galactosidase, catalase, tryptic soy digests, tryptose, lectins, E. coli single-stranded binding (SSB) protein, phage T4 gene 32 protein, and the like, or fragments or derivatives thereof. Examples of non-polypeptide additives include, but are not limited to; tRNA, rRNA, sulfur-containing compounds, acetate-containing compounds, dithiothreitol (DTT), dimethylsulfoxide (DMSO), glycerol, formamide, betain, tetramethylammonium chloride (TMAC), polyethylene glycol (PEG), TWEEN 20 non-ionic surfactant, NP 40, non- ionic surfactant, ectoine, and polyols. Preferred additives include DTT, DMSO, glycerol, formamide, betain, TMAC, PEG, TWEEN 20 non-ionic surfactant, NP 40 non-ionic surfactant, ectoine, polyols, E. coli (SSB) protein, Phage T4 gene 32 protein, and BSA. Particularly preferred additives include DTT, glycerol, Phage T4 gene 32 protein, and BSA.

In addition, amplification can be performed in the presence of agents that provide a means for detection of the amplification products. For example, the reaction vessel can already contain appropriate hybridization probes for homogenous real time detection of amplification products. Preferably, these probes can be appropriately labeled with fluorescent moieties. Other components include dyes that bind to double-stranded DNA. In some embodiments, the dye can be SYBR green. One of skill in the art will appreciate that other dyes are useful in the present invention.

### Homopolymeric Nucleic Acids

Homopolymeric nucleic acids have been found to serve as an inhibition-relieving agent capable of suppressing, or otherwise reducing, the inhibition of nucleic acid replication by reverse transcriptase, as observed in RT-PCR. A homopolymeric nucleic acid is any oligonucleotide of two or more bases in length composed of a single nucleotide species. Homopolymeric nucleic acids may be ribonucleic acids or deoxyribonucleic acids. Preferred homopolymeric ribonucleic acids include poly(A), poly(C), poly(G) or poly(U). Preferred homopolymeric deoxyribonucleic acids include poly(dA), poly(dC), poly(dG), or poly(dI). Homopolymer optionally used for RT-PCR is an oligonucleotide of two or more bases in length, preferably between 100 to 5000 bases in length, and more preferably between 200 to 2000 bases in length. An optional amount of nucleic acid homopolymer used for RT-PCR may be greater than 10 ng, preferably between 10 ng to 2000 ng, more preferably between 100 ng to 1000 ng, and most preferably between 200 ng to 600 ng. In certain embodiments a final concentration of nucleic acid homopolymer in an RT-PCR is between 200 ng/ml to 40000 ng/ml, preferably between 2000 ng/ml to 20000 ng/ml, and more preferably between 4000 ng/ml to 12000 ng/ml.

### Reagent kits for RT-PCR

A further aspect of the present invention is a reagent kit for RT-PCR, comprising at least one reverse transcriptase, optionally at least one DNA polymerase, and at least one buffer composition. In such a reagent kit, said reverse transcriptase, and optional DNA polymerase may be combined in one container and the buffer composition in a second container. Alternatively, a reverse transcriptase and optional DNA polymerase may be in one container with the buffer composition in a single container. In another example, a reverse transcriptase and a buffer composition may be combined in one container and an optional DNA polymerase may be in a second container. In some examples all three components are in separate containers. Such reagent kit may further comprise a mixture of two or more of dATP, dCTP, dGTP, and dTTP, and/or one or more additives useful in RT-PCR and/or one or more oligonucleotide primers. All components may be in separate containers, or two or more of them may be combined in one container. Preferably, where two enzymes are present, the enzymes are combined in one container, either together with a buffering agent, and/or a buffering agent is combined in a separate container with oligonucleotide primers, a dNTP mix, and one or more useful additives. Useful additives, if present, may be provided in the kit in separate containers.

In some examples, provided compositions comprise a mixture of random hexamer primer nucleic acids, a buffering agent comprising Tris, a magnesium chloride salt, an ammonium sulfate salt, deoxyribonucleoside triphosphates, DTT, and glycerol. In certain examples compositions comprise about 24ng/uL random hexamer, about 25mM Tris, about 4mM magnesium chloride, about 10mM ammonium sulfate, about 1mM dNTP, about 1mM DTT, about 4.4% glycerol and optionally reverse transcriptase. In other certain examples compositions comprise about 120ng/uL random hexamer, about 125mM Tris, about 20mM magnesium chloride, about 50 ammonium sulfate, about 5mM dNTP, about 5mM DTT, about 22% glycerol.

In certain examples kits are provided are provided for use in RT-PCR comprising compositions of the invention. In some examples a reverse transcriptase, and optionally a DNA polymerase and are combined in one container, and a mixture of random hexamer primer nucleic acids, a buffering agent comprising Tris, a magnesium chloride salt, an ammonium sulfate salt, deoxyribonucleoside triphosphates, DTT, and glycerol are combined in a second container. In some examples a reverse transcriptase, and optionally a DNA polymerase and are combined in one container, and a mixture of random hexamer primer nucleic acids, a buffering agent comprising Tris, a magnesium chloride salt, an ammonium sulfate salt, deoxyribonucleoside triphosphates, DTT, and glycerol are combined in a second container. In certain examples a kit comprises SuperScript IV RT Enzyme mix in one container and a mixture comprising about 120ng/uL random hexamer, about 125mM Tris, about 20mM magnesium chloride, about 50 ammonium sulfate, about 5mM dNTP, about 5mM DTT, about 22% glycerol in a second container.

### EXEMPLIFICATION

### Example 1:

Reaction compositions were prepared according to Table 1.

RT and library preparation were carried out using an AmpliSeq HD custom panel on two different cell line samples in control and two reactions with different preparations (a/b) of URT, followed by sequencing analysis on an Ion S5. Use of URT resulted in reduced primer dimer and increase in productive and on target reads; however did not result in significant effects of decrease on uniformity or family number. See Table 2. Similar results were seen with high and low plexy panels, using both AmpliSeq technology workflow or AmpliSeq HD technology workflow according to manufacturer instructions (data not shown). Additionally, similar results with improved effects were demonstrated on both manual and automated workflows. Notably, panels having increased primer dimer effects yielded a higher productive impact with inclusion of URT buffer over those reactions having little primer dimer. When individual components of URT buffer were added, whether individually or in sub-combinations, none yielded positive results of URT composition as a whole. Bioanalyzer traces show little to no primer dimer with use of either lot preparations of URT (data not shown).

**TABLE 1: Compositions**

| **Component** | **5x concentration** | **1× concentration** | **1× concentration** | **1× concentration** |
|---|---|---|---|---|
| TrisHCl ph8.4 | 125mM | 25mM | 25mM | 25mM |
| Ammonium sulfate | 50mM | 10mM | 10mM | 10mM |
| MgCl2 | 20mM | 4mM | 4mM | 4mM |
| dNTP pH7.6 | 5mM | 1mM | 1mM | 1mM |
| DTT | 5mM | 1mM | 1mM | 1mM |
| H2O | 34% | 6.8% | 6.8% | 6.8% |
| Glycerol | 22% | 4.44% | 4.44% | 4.44% |
| Random Hexamer | 120ng/uL | 24ng/uL | 24ng/ul | 24ng/ul |
| 5x Ion AmpliSeq Master Mix | - | - | 1× | - |
| 4x Ion AmpliSeq HD Master Mix | - | - | - | 1× |
| RT enzyme mix (SuperScriptIV (200U/uL) | - | - | 10U | 10U |

**TABLE 2**

| **Sample and condition** | 10ngDNA HEv1.1-DNA-RNA control | 10ngDNA HEv1.1-DNA-RNA URTa | 10ngDNA HEv1.1-DNA-RNA URTb | 5ng NA24385 control | 5ng NA24385 URTa | 5ng NA24385 URTb |
|---|---|---|---|---|---|---|
| **% primer dimer** | 26.27 | 6.14 | 7.77 | 13.69 | 5.16 | 1.83 |
| **% productive reads** | 91.60% | 95.20% | 94.50% | 65.02% | 82.62% | 89.41% |
| **% on target reads** | 94.7% | 96.60% | 96.20& | 76.11% | 87.53% | 91.42% |
| **Uniformity** | 96.90% | 96.70% | 96.90% | 98.2% | 98.66% | 98.44% |
| **Mean depth per target** | 64468 | 15670 | 21692 | 13021 | 23154 | 56483 |
| **Median # Family** | 2201 | 1715 | 1850 | 769 | 781 | 1427 |

**TABLE A: RT Reaction mix**

| **Component** | **Volume** |
|---|---|
| 20 ng input cfTNA/10 ng RNA | 15 µL |
| 5x URT reaction buffer | 4 µL |
| 10× RT Enzyme Mix (SSIV) | 2 µL |
| **Total volume** | **21 µL** |

**TABLE B: RT Reaction conditions**

| **Stage** | **Temperature** | **Time** |
|---|---|---|
| Stage 1 | 25°C | 10 min |
| Stage 2 | 50°C | 10 min |
| Stage 3 | 85°C | 5 min |
| Hold | 4°C | ∞ |

Example 2: Conversion of RNA to cDNA using URT is about equivalent to digital PCR.

NGS RT and library preparation was carried out according to Example 1 above. Gene fusions were also detected in parallel prepared sample by digital PCR and results compared. NGS RT, library preparation and sequencing compared to detection of fusions by digital PCR demonstrated similar results as measured by conversion rate when family numbers identified through URT/NGS method were compared to molecules detected by digital PCR. See Table 3.

**TABLE 3**

| **Fusion** | **URT Family number** | **Digital PCR copy #** |
|---|---|---|
| CD74-ROS1 | 578 | 713 |
| ETV6-NTRK3 | 1027 | 703 |
| FGFR3-TACC3 | 541 | 431 |
| KIF5B-RET | 1419 | 1033 |
| NCOA4-RET | 670 | 638 |
| SLC34A2-ROS1 | 256 | 656 |
| SLC45A3-BRAF | 523 | 643 |
| TPM3-NTRK1 | 693 | 733 |
| **Total family number** | **5707** | **5550** |
| **% conversion rate** | **103** | |

| | | |
|---|---|---|
| (conversion rate = family number/digital copy #) | | |

### Example 3: URT efficiently converts RNA-to cDNA

- NGS RT and library preparation was carried out according to Example 1 above, except a parallel preparation was carried out using SuperScript III reaction components in lieu of URT according to product instruction. Use of URT outperforms SuperScript III on assay RNA content. Yield and family number are improved when URT is used as compared to SuperScript III. The AmpliSeq HD library yield using URT resulted in 3-5 fold more library yield than SuperScript III and URT resulted in 2-3 fold more family generation than SuperScript III. URT thus resulted in improved conversion rate than SuperScript III. See Table 4 and Table 5.

**TABLE 4**

| FFPE samples | Library Yield (pM) | |
|---|---|---|
| | SSIII | URT |
| AD 139 | 74 | 223 |
| | 72 | 316 |
| NA1261 | 33 | 95 |
| | 16 | 75 |
| BC1007 | 308 | 1370 |
| | 254 | 1893 |

**TABLE 5**

| FFPE samples | Fusion Family Number | |
|---|---|---|
| | SSIII | URT |
| AD139 | 39 | 112 |
| | 12 | 123 |
| NA1261 | 17 | 44 |
| | 27 | 48 |
| BC1007 | 62 | 262 |
| | 74 | 282 |

Products (5ul or 10ul) from RT reactions using SuperScript IV VILO or URT reaction were prepared for analysis using the ONCOMINE Myeloid Panel according to manufacturer instructions and sequences on an ION S5. Using SuperScript IV VILO in the RT reaction, good performance can be achieved only when 5ul of the RT reaction is used. Longer fusion reads are greatly reduced when more RT reaction is used for library preparation and sequencing. When sensitivity is required for any application, such results could drastically impact the outcome and using half the RT reaction is not an option for any high sensitivity application. In contrast, fusion reads were not impacted when URT was utilized in RT reactions. See Table 6.

**TABLE 6**

| | **Fusion reads/million (N=3)** | | | | |
|---|---|---|---|---|---|
| | **VILOIV** | | **URT** | | **Insert length (bp)** |
| **Fusion** | 5uL | 10uL | 5uL | 10uL | |
| FIP1L1-PDGFRA. | 11703 | 13668 | 13191 | 14980 | 70 |
| TCF3-PBX1 | 6869 | 6483 | 7720 | 7629 | 71 |
| KAT6A-CREBP | 6825 | 6599 | 8193 | 8391 | 95 |
| PCM1-JAK2 | 9425 | 7703 | 12908 | 12028 | 99 |
| BCR-ABL | 5465 | 6405 | 8192 | 8114 | 102 |
| ETV6-ABL | 7457 | 6206 | 9402 | 8416 | 117 |
| RUNX1-RUNX1T1 | 4896 | 4038 | 5068 | 4499 | 127 |
| ETV6-ABL | 4423 | 865 | 6727 | 4635 | 133 |
| PML-RARA | 3592 | 936 | 4146 | 3524 | 250 |

While preferred embodiments of the present invention have been shown and described herein, it will be obvious to those skilled in the art that such embodiments are provided by way of example only. Numerous variations, changes, and substitutions will now occur to those skilled in the art without departing from the invention.

## Claims

1. A composition suitable for reverse transcription-polymerase chain reaction (RT-PCR) of RNA, comprising a mixture of oligonucleotide primer nucleic acids, a buffering agent comprising Tris, a magnesium chloride salt, an ammonium sulfate salt, deoxyribonucleoside triphosphates, and additives that are useful in RT-PCR,
wherein the additives useful in RT-PCR comprise DTT and glycerol,
wherein the oligonucleotide primer nucleic acids comprise random primers, primers specific to a target RNA template, and/or homopolymer primers, or a mixture of two or more of the foregoing, and wherein the composition provides for the preparation of cDNA from RNA for multiplexed libraries suitable for downstream analysis.

2. The composition of claim 1, wherein the composition contains at least 10 ng or at least 100 ng/ml of oligonucleotide primer, preferably wherein the composition contains between 10 to 2000 ng or between 100 and 10000 ng/ml of oligonucleotide primer.

3. The composition of claim 1, further comprising at least one member of the group consisting of a reverse transcriptase, wherein the reverse transcriptase is AMV-RT, M-MLV-RT, HIV-RT, EIAV-RT, RAV2-RT, C. hydrogenoformans DNA Polymerase, SuperScript I, SuperScript II, SuperScript III, SuperScript IV and/or mutants, variants, and derivatives thereof.

4. The composition of any one of claims 1 to 3, further comprising a DNA polymerase, wherein the DNA polymerase is Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT^{™}, DEEPVENT^{™} DNA polymerase, and/or mutants, variants, and derivatives thereof.

5. The composition of any one of claims 1 to 4, wherein the buffering agent comprises Tris at pH of about 8 to about 9.

6. The composition of any one of claims 1 to 5 further comprising an additive useful in RT-PCR preferably being DMSO, formamide, betain, tetramethylammonium chloride, PEG, Tween 20, NP 40, ectoine, polyoles, E. coli SSB protein, Phage T4 gene 32 protein, and/or BSA.

7. The composition of any one of claims 1 to 6 wherein the nucleotide triphosphates preferably are selected from dATP, dCTP, dGTP, dTTP, dITP, dUTP, α-thio-dNTPs, biotin-dUTP, fluorescein-dUTP, and/or digoxigenin-dUTP.

8. The composition of any one of claims 1 to 7 comprising about 24 ng/uL random hexamer, about 25 mM Tris, about 4 mM magnesium chloride, about 10 mM ammonium sulfate, about 1 mM dNTP, about 1 mM DTT, about 4.4 % glycerol and optionally reverse transcriptase or
about 120 ng/uL random hexamer, about 125 mM Tris, about 20 mM magnesium chloride, about 50 mM ammonium sulfate, about 5 mM dNTP, about 5 mM DTT, about 22 % glycerol.

9. A reagent kit for RT-PCR, comprising the composition of any one of claims 1-8,
wherein a reverse transcriptase, and optionally a DNA polymerase and are combined in one container, and a mixture of random hexamer primer nucleic acids, a buffering agent comprising Tris, a magnesium chloride salt, an ammonium sulfate salt, deoxyribonucleoside triphosphates, DTT, and glycerol are combined in a second container.

10. Use of a composition according to any one of claims 1 to 8 or a reagent kit according to claim 9 for RT-PCR.

11. A method for generating a nucleic acid from an RNA template comprising:
a) providing a sample comprising an RNA template to a reaction mixture, the reaction mixture comprising at least one reverse transcriptase, optionally at least one nucleic acid polymerase, a mixture of oligonucleotide primer nucleic acids, a buffering agent comprising Tris, a magnesium chloride salt, an ammonium sulfate salt, deoxyribonucleoside triphosphates, and additives that are useful in RT-PCR; and
b) incubating the reaction mixture under conditions sufficient to allow polymerization of a nucleic acid molecule complementary to a portion of the RNA template,
wherein the additives useful in RT-PCR comprise DTT and glycerol, wherein the oligonucleotide primer nucleic acids comprise random primers, primers specific to a target RNA template, and/or homopolymer primers, or a mixture of two or more of the foregoing, and wherein the composition provides for the preparation of cDNA from RNA for multiplexed libraries suitable for downstream analysis.

12. The method of claim 11, further comprising amplification of the cDNA molecule complementary to a portion of the RNA template by a process of DNA replication comprising a polymerase chain reaction (PCR).

13. The method of claim 12, wherein the reactions of DNA generation and amplification occur in an addition only uncoupled reaction mixture.

14. The method of claim 12 or 13, wherein reverse transcription occurs between about 35 °C to about 70 °C and the DNA replication comprises PCR amplification thermocycling.

15. The method of claim 14, wherein PCR amplification comprises at least 2 or 3 cycles of denaturation, annealing, and polymerization, or preferably wherein PCR amplification comprises at least 15 cycles of denaturation, annealing, and polymerization.

16. The method of any one of claims 12-15, wherein PCR amplification comprises multiplex amplification, preferably wherein PCR amplification comprises multiple different PCR amplification rounds.

## Patentansprüche

1. Zusammensetzung, die für die reverse Transkription-Polymerase-Kettenreaktion (RT-PCR) von RNA geeignet ist, umfassend eine Mischung aus Oligonukleotid-Primer-Nukleinsäuren, ein Puffermittel, umfassend Tris, ein Magnesiumchloridsalz, ein Ammoniumsulfatsalz, Desoxyribonukleosidtriphosphate und Additive, die bei der RT-PCR nützlich sind,
wobei die Additive, die in der RT-PCR nützlich sind, DTT und Glycerin umfassen,
wobei die Oligonukleotid-Primer-Nukleinsäuren willkürliche Primer, Primer, die für eine Ziel-RNA-Vorlage spezifisch sind und/oder Homopolymer-Primer oder eine Mischung aus zwei oder mehr oder der Vorstehenden umfassen, und wobei die Zusammensetzung die Herstellung von RNA für cDNA für gemultiplexte Bibliotheken bereitstellt, die für eine nachgeschaltete Analyse geeignet sind.

2. Zusammensetzung nach Anspruch 1, wobei die Zusammensetzung mindestens zu 10 ng oder mindestens 100 ng/ml Oligonukleotid-Primer enthält, vorzugsweise wobei die Zusammensetzung zu zwischen 10 und 2000 ng oder zwischen 100 und 10000 ng/ml Oligonukleotid-Primer enthält.

3. Zusammensetzung nach Anspruch 1, ferner umfassend mindestens ein Mitglied der Gruppe bestehend aus einer reversen Transkriptase, wobei die reverse Transkriptase AMV-RT, M-MLV-RT, HIV-RT, EIAV-RT, RAV2-RT, C. hydrogenoformans DNA-Polymerase, SuperScript I, SuperScript II, SuperScript III, SuperScript IV und/oder Mutanten, Varianten und Derivate davon ist.

4. Verfahren nach einem der Ansprüche 1 bis 3, ferner umfassend eine DNA-Polymerase, wobei
die DNA-Polymerase Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT^{™}, DEEPVENT^{™} DNA-Polymerase und/oder Mutanten, Varianten und Derivate davon ist.

5. Zusammensetzung nach einem der Ansprüche 1 bis 4, wobei das Puffermittel Tris bei einem pH-Wert von etwa 8 bis etwa 9 umfasst.

6. Zusammensetzung nach einem der Ansprüche 1 bis 5, ferner umfassend ein für die RT-PCR nützliches Additiv, bei dem es sich vorzugsweise um DMSO, Formamid, Betain, Tetramethylammoniumchlorid, PEG, Tween 20, NP 40, Ectoin, Polyole, E. coli SSB-Protein, Phage T4 Gen 32-Protein und/oder BSA handelt.

7. Zusammensetzung nach einem der Ansprüche 1 bis 6, wobei die Nukleotidtriphosphate vorzugsweise ausgewählt sind aus dATP, dCTP, dGTP, dTTP, dITP, dUTP, α-thio-dNTPs, Biotin-dUTP, Fluorescein-dUTP und/oder Digoxigenin-dUTP.

8. Zusammensetzung nach einem der Ansprüche 1 bis 7, etwa 24 ng/uL willkürliches Hexamer, etwa 25 mM Tris, etwa 4 mM Magnesiumchlorid, etwa 10 mM Ammoniumsulfat, etwa 1 mM dNTP, etwa 1 mM DTT, etwa 4,4 % Glycerin und optional Reverse Transkriptase oder
etwa 120 ng/uL willkürliches Hexamer, etwa 125 mM Tris, etwa 20 mM Magnesiumchlorid, etwa 50 Ammoniumsulfat, etwa 5 mM dNTP, etwa 5 mM DTT, etwa 22 % Glycerin.

9. Reagenzkit für die RT-PCR, umfassend die Zusammensetzung nach einem der Ansprüche 1 bis 8,
wobei eine Reverse Transkriptase und optional eine DNA-Polymerase und in einem Behälter kombiniert werden, und eine Mischung aus willkürlichen Hexamer-Primer-Nukleinsäuren, ein Puffermittel, umfassend Tris, ein Magnesiumchloridsalz, ein Ammoniumsulfatsalz, Desoxyribonukleosidtriphosphate, DTT und Glycerin in einem zweiten Behälter kombiniert werden.

10. Verwendung einer Zusammensetzung nach einem der Ansprüche 1 bis 8 oder eines Reagenzkits nach Anspruch 9 für die RT-PCR.

11. Verfahren zum Erzeugen einer Nukleinsäure aus einer RNA-Vorlage, umfassend:
a) Bereitstellen einer Probe, umfassend eine RNA-Vorlage an eine Reaktionsmischung, die Reaktionsmischung umfassend mindestens eine Reverse Transkriptase, optional mindestens eine Nukleinsäurepolymerase, eine Mischung aus Oligonukleotid-Primer-Nukleinsäuren, ein Puffermittel, umfassend Tris, ein Magnesiumchloridsalz, ein Ammoniumsulfatsalz, Desoxyribonukleosidtriphosphate und Additive, die bei der RT-PCR nützlich sind; und
b) Inkubieren der Reaktionsmischung unter Bedingungen, die ausreichen, um eine Polymerisation eines Nukleinsäuremoleküls zu ermöglichen, das zu einem Abschnitt der RNA-Vorlage komplementär ist,
wobei die in der RT-PCR nützlichen Additive DTT und Glycerin umfassen, wobei die Oligonukleotid-Primer-Nukleinsäuren willkürliche Primer, Primer, die für eine Ziel-RNA-Vorlage spezifisch sind und/oder Homopolymer-Primer oder eine Mischung aus zwei oder mehr oder der Vorstehenden umfassen, und wobei die Zusammensetzung die Herstellung von RNA für cDNA für gemultiplexte Bibliotheken bereitstellt, die für die nachgeschaltete Analyse geeignet sind.

12. Verfahren nach Anspruch 11, ferner umfassend Amplifikation des cDNA-Moleküls, das komplementär zu einem Abschnitt der RNA-Vorlage ist, durch einen Vorgang der DNA-Replikation, umfassend eine Polymerase-Kettenreaktion (PCR).

13. Verfahren nach Anspruch 12, wobei die Reaktionen der DNA-Erzeugung und -Amplifikation in einer zusätzlichen, nur ungekoppelten Reaktionsmischung erfolgt.

14. Verfahren nach Anspruch 12 oder 13, wobei eine Reverse Transkription bei zwischen etwa 35 °C bis etwa 70 °C erfolgt und die DNA-Replikation PCR-Amplifikations-Thermozyklen umfasst.

15. Verfahren nach Anspruch 14, wobei die PCR-Amplifikation mindestens 2 oder 3 Zyklen der Denaturierung, des Temperns und der Polymerisation umfasst, oder vorzugsweise, wobei die PCR-Amplifikation mindestens 15 Zyklen der Denaturierung, des Temperns und der Polymerisation umfasst.

16. Verfahren nach einem der Ansprüche 12 bis 15, wobei die PCR-Amplifikation eine Multiplex-Amplifikation umfasst, vorzugsweise wobei die PCR-Amplifikation vielfache unterschiedliche PCR-Amplifikationsrunden umfasst.

## Revendications

1. Composition appropriée pour une réaction en chaîne de la polymérase par transcription inverse (RT-PCR) d'un ARN, comprenant un mélange d'acides nucléiques d'amorce oligonucléotidiques, un agent tampon comprenant du Tris, un sel de chlorure de magnésium, un sel de sulfate d'ammonium, des triphosphates de désoxyribonucléoside, et des additifs qui sont utiles dans une RT-PCR,
dans laquelle les additifs utiles dans la RT-PCR comprennent DTT et glycérol,
dans laquelle les acides nucléiques d'amorce oligonucléotidiques comprennent des amorces aléatoires, des amorces spécifiques à une matrice d'ARN cible, et/ou des amorces homopolymères, ou un mélange d'au moins deux ou de ce qui précède, et dans laquelle la composition fournit la préparation d'un ARN à l'ADNc pour des banques multiplexées appropriées pour une analyse en aval.

2. Composition selon la revendication 1, dans laquelle la composition contient au moins 10 ng ou au moins 100 ng/ml d'amorce oligonucléotidique, de préférence dans laquelle la composition contient entre 10 et 2000 ng ou entre 100 et 10 000 ng/ml d'amorce oligonucléotidique.

3. Composition selon la revendication 1, comprenant en outre au moins un membre du groupe constitué d'une transcriptase inverse, dans laquelle la transcriptase inverse est AMV-RT, M-MLV-RT, HIV-RT, EIAV-RT, RAV2-RT, ADN Polymérase C. hydrogénoformans, SuperScript I, SuperScript II, SuperScript III, SuperScript IV et/ou mutants, variants et dérivés de ceux-ci.

4. Composition selon l'une quelconque des revendications 1 à 3, comprenant en outre une ADN polymérase, dans laquelle
l'ADN polymérase est Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT^{™}, DEEPVENT^{™} ADN polymérase, et/ou mutants, variantes et dérivés de ceux-ci.

5. Composition selon l'une quelconque des revendications 1 à 4, dans laquelle l'agent tampon comprend du Tris à un pH d'environ 8 à environ 9.

6. Composition selon l'une quelconque des revendications 1 à 5, comprenant en outre un additif utile dans la RT-PCR étant de préférence DMSO, formamide, bétaïne, chlorure de tétraméthylammonium, PEG, Tween 20, NP 40, ectoïne, polyols, protéine SSB de E. coli, protéine 32 de gène T4 phage et/ou BSA.

7. Composition selon l'une quelconque des revendications 1 à 6, dans laquelle les nucléotides triphosphates sont de préférence choisis parmi dATP, dCTP, dGTP, dTTP, dITP, dUTP, α-thio-dNTPs, biotine-dUTP, fluorescéine-dUTP et/ou digoxigénine-dUTP.

8. Composition selon l'une quelconque des revendications 1 à 7 comprenant environ 24 ng/uL d'hexamère aléatoire, environ 25 mM de Tris, environ 4 mM de chlorure de magnésium, environ 10 mM de sulfate d'ammonium, environ 1 mM de dNTP, environ 1 mM de DTT, environ 4,4 % de glycérol et éventuellement une transcriptase inverse ou
environ 120 ng/uL d'hexamère aléatoire, environ 125 mM de Tris, environ 20 mM de chlorure de magnésium, environ 50 sulfate d'ammonium, environ 5 mM de dNTP, environ 5 mM de DTT, environ 22 % de glycérol.

9. Kit de réactif pour la RT-PCR, comprenant la composition selon l'une quelconque des revendications 1 à 8,
dans lequel une transcriptase inverse, et éventuellement une ADN polymérase et sont combinées dans un récipient, et un mélange d'acides nucléiques d'amorce d'hexamère aléatoire, d'un agent tampon comprenant du Tris, d'un sel de chlorure de magnésium, d'un sel de sulfate d'ammonium, des triphosphates de désoxyribonucléoside, du DTT, et du glycérol sont combinés dans un second récipient.

10. Utilisation d'une composition selon l'une quelconque des revendications 1 à 8 ou d'un kit de réactif selon la revendication 9 pour la RT-PCR.

11. Procédé de génération d'un acide nucléique à partir d'un modèle d'ARN comprenant :
a) la fourniture d'un échantillon comprenant une matrice d'ARN à un mélange réactionnel, le mélange réactionnel comprenant au moins une transcriptase inverse, éventuellement au moins une polymérase d'acide nucléique, un mélange d'acides nucléiques d'amorce oligonucléotidique, un agent tampon comprenant du Tris, un sel de chlorure de magnésium, un sel de sulfate d'ammonium, des triphosphates de désoxyribonucléoside et des additifs qui sont utiles dans une RT-PCR ; et
b) l'incubation du mélange réactionnel dans des conditions suffisantes pour permettre la polymérisation d'une molécule d'acide nucléique complémentaire d'une partie du modèle d'ARN,
dans lequel les additifs utiles dans la RT-PCR comprennent du DTT et du glycérol, dans lequel les acides nucléiques d'amorce oligonucléotidique comprennent des amorces aléatoires, des amorces spécifiques à une matrice d'ARN cible, et/ou des amorces homopolymères, ou un mélange d'au moins deux ou de ce qui précède, et dans laquelle la composition fournit la préparation d'ARN à l'ADNc pour des banques multiplexées appropriées pour une analyse en aval.

12. Procédé selon la revendication 11, comprenant en outre l'amplification de la molécule d'ADNc complémentaire d'une partie de la matrice d'ARN par un processus de réplication d'ADN comprenant une réaction en chaîne par polymérase (PCR).

13. Procédé selon la revendication 12, dans lequel les réactions de génération d'ADN et d'amplification se produisent en addition uniquement du mélange réactionnel découplé.

14. Procédé selon la revendication 12 ou 13, dans lequel la transcription inverse se produit entre environ 35 °C et environ 70 °C et la réplication d'ADN comprend un thermocyclage d'amplification par PCR.

15. Procédé selon la revendication 14, dans lequel l'amplification par PCR comprend au moins 2 ou 3 cycles de dénaturation, de recuit et de polymérisation, ou de préférence dans lequel l'amplification PCR comprend au moins 15 cycles de dénaturation, de recuit et de polymérisation.

16. Procédé selon l'une quelconque des revendications 12 à 15, dans lequel l'amplification PCR comprend une amplification multiplexe, de préférence dans lequel l'amplification PCR comprend de multiples cycles d'amplification PCR différents.
